(19) Europäisches Patentamt
European Patent Office
Office européen des brevets

(11) Veröffentlichungsnummer **0 012 868**
**A1**

(12) # EUROPÄISCHE PATENTANMELDUNG

(21) Anmeldenummer: 79104758.2

(22) Anmeldetag: 29.11.79

(51) Int. Cl.³: C 07 D 285/06
C 07 F 1/00, C 07 F 3/00

(30) Priorität: 28.12.78 DE 2856404

(43) Veröffentlichungstag der Anmeldung:
09.07.80 Patentblatt 80/14

(84) Benannte Vertragsstaaten:
BE CH DE FR GB IT NL

(71) Anmelder: DYNAMIT NOBEL AKTIENGESELLSCHAFT
Patentabteilung Postfach 1209
D-5210 Troisdorf, Bez. Köln(DE)

(72) Erfinder: Bison, Günter
Kleiststrasse 6
D-521 Troisdorf-Sieglar(DE)

(72) Erfinder: Breideneichen, Kurt
Am Hang 14
D-521 Troisdorf-Spich(DE)

(72) Erfinder: Wolfes, Wolfgang, Dr.
Bahnstrasse 11
D-5216 Niederkassel-Mondorf(DE)

(54) **1,2,3-Thiadiazol-5-yl-thioglycolsäure, deren Derivate sowie Verfahren zu deren Herstellung.**

(57) Verbindungen der Formel

I,

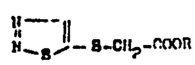

worin R ein Wasserstoffatom, ein Metallatom der 1. oder 2. Hauptgruppe, Ammonium oder einen Alkylrest mit 1 bis 8 C-Atomen bedeutet,
Metallverbindungen der Formel

II,

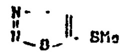

worin Me ein Metallatom der 1. oder 2. Hauptgruppe oder Ammonium bedeutet sowie deren Herstellung.

EP 0 012 868 A1

Troisdorf, den 1. Juni 1979
OZ: 78 102 ( 2924 )

DYNAMIT NOBEL AKTIENGESELLSCHAFT
Troisdorf, Bez. Köln

# 1,2,3-Thiadiazol-5-yl-thioglycolsäure, deren Derivate sowie Verfahren zu deren Herstellung

Die vorliegende Erfindung betrifft 1,2,3-Thiadiazol-5-yl-thioglycolsäure, deren Derivate und Verfahren zu ihrer Herstellung.
Die Erfindung betrifft weiter die zur Herstellung dienenden Thiadiazol-5-metallthiole.

Die erfindungsgemäßen Verbindungen entsprechen der Formel I

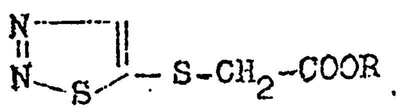

I,

worin R ein Wasserstoffatom, ein Metallatom oder einen Alkylrest, mit 1 bis 8 C-Atomen bedeutet.
Alkylreste mit 1 bis 4 C-Atomen sind bevorzugt.
Bevorzugt sind als Metallatome die Elemente der 1. und 2. Hauptgruppe sowie Ammonium, von denen bevorzugt Natrium, Kalium, Lithium, Ammonium und ggf. Calcium zu nennen sind.
Als Alkylreste können beispielsweise Methyl, Aethyl, n-Propyl, iso-Propyl, n-, iso-, sec-oder ter.-Butyl, Pentyl, Hexyl, Heptyl und Octyl eingesetzt werden.

Die Verbindungen der Formel I können durch Umsetzung von Verbindungen der Formel

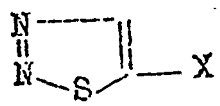 III,

worin X Halogen bedeutet, mit Thioglycolsäure, deren Estern oder Salzen hergestellt werden. Halogen ist bevorzugt Chlor, gegebenenfalls Brom.
Die Gegenwart von säurebindenden Mitteln ist bevorzugt. die in molarer Menge oder gegebenenfalls einem Überschuß bis etwa 10 Mol % verwendet werden.
Als säurebindende Mittel kommen Verbindungen wie z.B. Alkalicarbonate oder Acetate, tertiäre Amine, vor allem aber Alkalialkoholate wie z.B. Natriumalkoholat,in Frage.
Die Ausgangsstoffe der Formel III sind bekannt und werden beispielsweise hergestellt, indem man ein Acylhydrazon der Formel

$$X - CH_2 - CH = N - NH - COR \qquad IV,$$

worin beispielsweise X Chlor bedeutet mit Thionylchlorid entsprechend der DE-OS 26 36 994 umsetzt.
Weiterer Gegenstand der Erfindung sind die Verbindungen der Formel

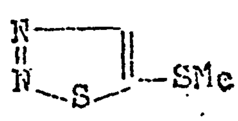 II,

worin Me ein Metallatom oder Ammonium bedeutet.

Das Metall Me kann ein an sich beliebiges Metall oder Ammonium sein, beispielsweise die oben bereits genannten Metalle der 1. oder 2. Hauptgruppe, jedoch wird aus praktischen Gründen sehr bevorzugt Natrium, Kalium oder $NH_4$ verwendet.

Metallsulfide der jeweils gewünschten Metalle bilden bei Umsetzung mit Stoffen der Formel III die Stoffe der Formel II. Temperaturen von 20°C bis 120°C sind zweckmäßig. Ein molarer Überschuß des Metallsulfids ist notwendig, wobei 0,5 Mol bis 2,0 Mol je Mol der Verbindung III zweckmäßig sind.
Je Mol Metallsulfid können zweckmäßig weiter 0,5 bis 2,0 Mol Schwefel anwesend sein. Ein Lösungsmittel aus der Gruppe der zur Herstellung der Verbindung I verwendeten Lösungsmittel ist anwesend, welches in vielen Fällen das später bei der Herstellung der Verbindungen I verwendete Lösungsmittel ist.

Im allgemeinen erfolgt die Reaktion in einem Lösungsmittel. Zweckmäßig sind Alkohole, beispielsweise Methanol, Aethanol und Isopropanol, gegebenenfalls auch andere polare Lösemittel wie Dimethylformamid und Acetonitril.

Verbindungen der Formel I können durch Umsetzung von Verbindungen der Formel II mit Halogenessigsäure, deren Estern oder Salzen hergestellt werden.

Als Halogen ist Chlor sehr bevorzugt und Brom bevorzugt. Bei der Wahl, ob die freie Halogenessigsäure oder deren Ester oder deren Salz verwendet wird, ist, wie oben, die Gruppe R der herzustellenden Verbindung der Formel I maßgeblich.

Als Halogenessigsäure können Chlor- bzw. Bromessigsäure

verwendet werden. Die Estergruppe stellt einen $C_1$-$C_8$ Alkylrest dar, der bevorzugt aus einer $C_1$-$C_4$ Alkylgruppe bestehen kann.
Die Temperaturen betragen im allgemeinen 20 bis 120°C, bevorzugt 30 bis 90°C.
Die Drucke können im Bereich von 1 bis 3 bar liegen.

Die Herstellung der Verbindungen der Formel I aus Verbindungen der Formel II kann in Wasser, in einem mit Wasser mischbaren Lösungsmittel oder in wasserfreiem Lösungsmittel durchgeführt werden. Als Beispiele seien genannt Aether, Dioxan, Benzol, Dimethyl-Formamid, vor allem aber Alkanole wie Methanol, Aethanol.

Die beanspruchten Verbindungen sind wertvolle Zwischenstufen, beispielsweise zur Herstellung von optischen Aufhellern auf dem Textilsektor.

<u>Beispiel 1</u> ( Verbindung der Formel II )
Natriumsalz des 5-Mercapto-1,2,3-thiadiazol

240 g ( 1 Mol ) Natriumsulfid. 9 $H_2O$ wurden in 375 ml Isopropanol suspendiert, unter kräftigem Rühren auf 80°C erhitzt und anschließend 32 g Schwefel zugefügt. Zu der erhaltenen, klaren, rötlich gefärbten Lösung wurde innerhalb 30 Minuten eine Lösung von 60, 25 g ( 0,5 Mol ) 5-Chlor-1,2,3-thiadiazol, gelöst in 200 ml Isopropanol zugetropft und 2 Stunden am Rückfluß erhitzt. Danach wurde der Rührer abgestellt, die obere Phase abgezogen und auf 20°C gekühlt, wobei sich der Hauptteil des Natriumsalzes, des 5-Mercapto-1,2,3- thiadiazols kristallin abschied. Das isolierte Produkte wurde mit Isopropanol gewaschen und im Umlufttrockenschrank bei 40°C getrocknet.
Ausbeute 68 g ( 82 % d.Th. ).
Festpunkt 266-8°C unter Verpüffung.
Die Substanz enthält 2 Kristallwasser.
Die Elementaranalyse ergab $C_2H_5O_2N_2S_2Na$.

berechnet: C 13,63; H 2,86; N 15,90; S 36,40; Na 13,05
gefunden : C 13,77; H 2,23; N 16,00; S 36,05; Na 12,00.

In entsprechender Weise wird das Kaliumsalz, das Ammoniumsalz und das Calciumsalz hergestellt.

<u>Beispiel 2</u> ( Verbindung der Formel I )
1,2,3-Thiadiazol-5-yl-thioglykolsäure

46,0 g (0,49 Mol) Thioglykolsäure wurden in 165 ml Wasser suspendiert und unter Rühren langsam mit 61,3 g (0,50 Mol) Natriumcarbonat versetzt. Die entstandene

wasserklare Salzlösung vom pH-Wert 9,4 wurde auf 60 - 70°C erhitzt. Darauf wurde innerhalb 15 Minuten eine Lösung von 60,25 g ( 0,5 Mol ) 5-Chlor-1,2,3-thiadiazol, gelöst in 150 ml Aethanol, zugetropft und anschließend das Reaktionsgemisch 4 Stunden bei dieser Temperatur gerührt.
Aethanol wurde unter vermindertem Druck weitgehend abgezogen; hierbei kristallisierte bereits ein Teil des Produkts aus.
Der Rückstand wurde mit 165 ml Wasser versetzt, auf 20°C gekühlt und mit ca. 36 Gew.-%iger Salzsäure versetzt, bis ein pH-Wert von 0,5 erreicht war, wobei die Kristallisation bereits bei pH-Werten zwischen 4 und 3 begann. Das Kristallisat wurde isoliert, mit Wasser gewaschen und getrocknet.
Ausbeute 72,6 g ( 82,4 % d.Th. ).
Festpunkt 127-8°C.
Die Reinheit der Substanz wurde nach DC im Laufmittel Butanol/$NH_3$ 80 : 20 bestimmt. Es war nur ein Hauptfleck erkennbar.

Elementaranalyse: $C_4H_4O_2N_2S_2$, ( Molgewicht ber.: 176 )

berechnet: C 27,26; H 2,29; N 15,90 ; S 36,39
gefunden : C 27,72; H 1,83; N 16,07 ; S 35,52.

Das Molgewicht wurde massenspektrometrisch zu 176 bestimmt.

<u>Beispiel 3</u> ( Verbindung der Formel I )
1,2,3-Thiadiazol-5-yl-thioglykolsäureäthylester

Eine Mischung aus 50,40 g ( 0,36 Mol ) 5-Mercapto-1,2,3-thiadiazol-Natrium, 44,30 g ( 0,36 Mol ) Chloressigsäureäthylester und 300 g 50 Gew.-%iges wässeriges Aethanol wurden 5 Stunden unter Rückfluß auf Siedetemperatur (82-3°C) gehalten. Die Mischung wurde auf Raumtemperatur abgekühlt und durch Absaugen filtriert. Der Rückstand, der im wesentlichen aus Natriumchlorid bestand, wurde mit Aethanol gewaschen. Das Filtrat und das Waschäthanol wurden vereinigt und im Rotationsverdampfer im Wasserstrahlvakuum eingeengt. Der Destillationsrückstand, eine braune Flüssigkeit mit ausgeschiedenem Natriumchlorid, wurde mit 200 ml Aether versetzt, kräftig durchgeschüttelt, das Kochsalz filtriert und mit Aether nachgewaschen. Die Aetherauszüge wurden vereinigt, mit Natriumsulfat getrocknet. Nach Abziehen des Aethers wurde der Rückstand fraktioniert destilliert.

Der gewonnene 1,2,3-Thiadiazol-5-yl-thioglykolsäureäthylester hatte einen Siedepunkt von 153-4°C bei 1,7 mbar und einen Brechungsindex $n_D^{20}$ = 1,5629.
Die Reinheit nach GC betrug 99,59 %.
Die Ausbeute 71,8 g, entsprechend 97,6 % bezogen auf eingesetztes 5-Mercapto-1,2,3-thiadiazol-Natrium.

Elementaranalyse: $C_6H_8O_2N_2S_2$ ( Molgewicht ber.: 204 )

berechnet: C 35,28 %; H 3,95 %; O 15,67 %; N 13,71 %; S 31,39 %
gefunden : C 35,55 %; H 3,99 %; O 15,38 %; N 13,89 %; S 31,19 %.

Das Molekulargewicht der erhaltenen Verbindung wurde massenspektrometrisch zu 204 bestimmt.

Beispiel 4 ( Verbindung der Formel I )

Entsprechend Beispiel 3, jedoch unter Verwendung von

Chloressigsäuremethylester, anstelle von Chloressigsäureäthylester, wird der entsprechende 1,2,3-Thiadiazol-5-yl-thioglykolsäuremethylester erhalten und durch Elementaranalyse bestätigt.

Beispiel 5 ( Verbindungen der Formel I )

Die gemäß Beispiel 2 hergestellte 1,2,3-Thiadiazol-5-yl-thioglykolsäure wird in der Wärme in jeweils 1,02 Äquivalenten

a) wässriger 50 Gew.-%iger Natriumhydroxidlösung

b) wässriger 40 Gew.-%iger Kaliumhydroxidlösung und

c) wässriger 20 Gew.-%iger Ammoniumhydroxidlösung

zur Lösung gebracht. Nach Abdampfen des Wassers unter vermindertem Druck wurde a) das Natriumsalz, b) das Kaliumsalz und c) das Ammoniumsalz der 1,2,3,-Thiadiazol-5-yl-thioglykolsäure erhalten und durch Elementaranalyse bestätigt.

Dr.La/af

0012868

Patentansprüche:

1. Verbindungen der Formel

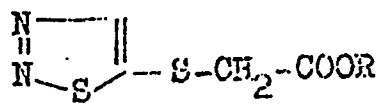

I,

worin R ein Wasserstoffatom, ein Metallatom der 1. oder 2. Hauptgruppe, Ammonium oder einen Alkylrest mit 1 bis 8 C-Atomen bedeutet.

2. Metallverbindungen der Formel

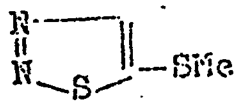

II,

worin Me ein Metallatom der 1. oder 2. Hauptgruppe oder Ammonium bedeutet.

3. Verfahren zur Herstellung von Verbindungen der Formel I durch Umsetzung von Metallverbindungen der Formel II mit Halogenessigsäure, deren Estern oder Salzen.

4. Verfahren zur Herstellung von Verbindungen der Formel I durch Umsetzung von Verbindungen der Formel

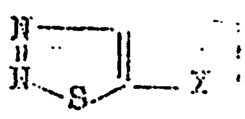

III,

worin X Halogen bedeutet, mit Thioglycolsäure, deren Estern oder Salzen.

5. Verfahren zur Herstellung von Verbindungen der Formel II durch Umsetzung von Verbindungen der Formel III mit Metallsulfiden und gegebenenfalls zusätzlich Schwefel, wobei das Metall der Metallsulfide

0012868

dem Metallatom Me der Formel II oder Ammonium entspricht.

Dr.La/af

0012868

Europäisches Patentamt

# EUROPÄISCHER RECHERCHENBERICHT

Nummer der Anmeldung

EP 79 10 4758

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl. 3) |
|---|---|---|---|
| X | JOURNAL OF HETEROCYCLIC CHEMISTRY, Band 15, Nr. 8, Dezember 1978, Provo, Utah, US, P. DEMAREE et al.: "Five-membered heterocyclic thiones. Part VII (1,2) 1,2,3-Thiadiazole-5-thiolates", Seiten 1295-1298<br>* Verbindungen 4d und 4c; Seite 1295; Seite 1297 und Seite 1298 *<br>---- | 1,2 | C 07 D 285/06<br>C 07 F 1/00<br>3/00 |

RECHERCHIERTE SACHGEBIETE (Int. Cl. 3)

C 07 D 285/06

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 28-03-1980 | CREMERS |